# FASCICULE DE BREVET EUROPEEN

(11) **EP 0 616 538 B1**
(45) Date de publication et mention de la délivrance du brevet: **21.04.1999**
(21) Numéro de dépôt: 93902285.1
(22) Date de dépôt: 03.12.1992
(51) Int. Cl.: A61K 49/04

(54) **NOUVEAU COMPOSE MACROMOLECULAIRE POLYAMINE IODE, SON PROCEDE DE PREPARATION ET SON UTILISATION COMME AGENT DE CONTRASTE**
NEUE JODIERTE MAKROMOLEKULARE POLYAMINVERBINDUNGEN, VERFAHREN ZUR HERSTELLUNG UND VERWENDUNG ALS KONTRASTMITTEL
NOVEL MACROMOLECULAR POLYAMINE IODINE-CONTAINING COMPOUND, PROCESS FOR ITS PREPARATION AND ITS USE AS A CONTRAST AGENT

(30) Priorité: 04.12.1991 FR 9115037; 13.03.1992 FR 9203063
(43) Date de publication de la demande: 28.09.1994
(73) Titulaire: GUERBET S.A., F-93430 Villepinte (FR)
(72) Inventeur: MEYER, Dominique, F-94100 Saint-Maur (FR); LE GRENEUR, Soizic, F-91440 Bures-sur-Yvette (FR)
(74) Mandataire: Koch, Gustave
(86) Numéro de dépôt international: FR9201135
(87) Numéro de publication internationale: WO9310824

(56) Documents cités:
- EP-A- 0 203 833
- EP-A- 0 354 836
- EP-A- 0 436 316
- WO-A-88/01178
- WO-A-88/01179
- WO-A-88/06162
- US-A- 4 406 878

## Description

L'invention a pour objet un nouveau composé macromoléculaire polyaminé iodé.

Elle vise également le procédé de préparation du susdit composé et son utilisation comme agent de contraste, notamment en radiologie aux rayons X.

Elle a enfin pour objet les compositions qui sont à base du susdit composé et qui sont administrables en vue d'un diagnostic.

On connaît déjà, par exemple par les brevets FR-A-87 01876 et FR-A-88 10794, des composés macromoléculaires iodés utilisables comme produits de contraste.

Ces composés donnent dans l'ensemble satisfaction du point de vue des propriétés bien connues de l'homme de l'art et requises pour permettre leur utilisation comme agents de contraste, à savoir notamment:
- une stabilité dans l'eau suffisamment élevée pour résister aux conditions de stérilisation,
- une concentration molaire élevée en iode,
- une osmolalité faible,
- une toxicité faible,
- une solubilité suffisante dans l'eau aux valeurs de pH physiologiquement acceptables.

Il n'en est pas moins vrai que leur préparation implique des contraintes qui se répercutent sur leur prix de revient.

Compte tenu de cet état de choses, la Société Demanderesse s'est donné comme but de mettre à la disposition des milieux médicaux des composés macromoléculaires répondant au moins aussi bien que ceux qui sont déjà connus aux exigences de la pratique en ce qui concerne les propriétés évoquées ci-dessus, tout en étant d'une préparation plus simple, enrichissant ainsi la panoplie d'agents de contraste offerts à l'utilisateur.

Elle a eu le mérite d'atteindre ce but grâce à la mise au point d'un composé macromoléculaire polyaminé d'origine biologique ou synthétique caractérisé par le fait qu'il porte au moins trois dérivés radio-opaques iodés.

Selon une forme de réalisation préférée de l'invention, le composé macromoléculaire polyaminé est un polymère de type dendrimère.

On désigne dans ce qui suit par le terme "dendrimères", des polymères comportant des ramifications et sur lesquels sont présents des groupes fonctionnels uniformément distribués à la périphérie desdites ramifications.

L'un des avantages principaux de ce type particulier de polymère est de pouvoir contrôler précisément leur taille et leur forme, en fonction de leur utilisation finale.

On désigne quelquefois ces polymères à poids moléculaire défini par le terme de polymère mono-dispersé (de l'anglais mono-dispersed polymer) par opposition aux polymères poly-dispersés (de l'anglais "poly-dispersed polymers") dont le poids moléculaire ne représente en fait qu'une moyenne des différents poids moléculaires présents dans le produit.

Pour la commodité du langage, on désignera dans ce qui suit par le terme "dendrimères", les polymères polyaminés également identifiés par les termes de polymères denses en étoile (de l'anglais "dense star polymers"), les polyaminés denses en étoile (de l'anglais "dense star polyamines"), les polymères éclatés en étoile (de l'anglais "Starburst polymers"), les polymères en cascade, les polyamines en étoile ramifiés en forme de peigne (de l'anglais "star combed-branched polyamines"), les polymères denses en étoile ramifiés (de l'anglais "bridged dense star polymers"), les arborols, les polylysines ramifiées.

Référence peut être faite pour les caractéristiques des dendrimères et arborols ainsi que pour leur procédé de préparation aux demandes de brevet et/ou brevets EP 271 180, US 4 507 466, US 4 694 064, US 4 713 975, US 4 857 599, US 4 690 985, US 4 599 400, US 4 871 779, US 4 737 550, US 4 587 329, US 4 568 737, US 4 558 120, US 4 631 337, ainsi qu'à la publication "Angewandte Chemie Int." Ed. Engl. 29 (1990), 138-175. Les polylysines ramifiées sont notamment décrites dans le brevet US 4 289 872 et dans la publication "Proc. Natl. Acad. Sci." USA, 85, 5409-5413, 1988.

Plus particulièrement, le composé macromoléculaire polyaminé iodé conforme à l'invention est caractérisé par le fait qu'il présente la formule générale:

P⁅NKₓ-A-G]ₙ (I)

dans laquelle
- P représente le reste d'un composé ou support macro-moléculaire polyaminé d'origine biologique ou synthétique,
- K représente un atome d'hydrogène ou un groupe alkyle inférieur linéaire ou ramifié, hydroxy- ou polyhydroxyalkyle inférieur linéaire ou ramifié, alcoxyalkyle inférieur linéaire ou ramifié, alcoxyhydroxy- ou alcoxypolyhydroxyalkyle inférieur linéaire ou ramifié, K pouvant également représenter un groupe -A-G,
- x est un nombre entier égal à 0 ou 1,
- G est un dérivé radio-opaque iodé constitué notamment par un dérivé benzénique de formule: 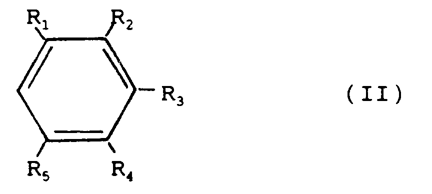 dans laquelle les constituants R₁, R₂, R₃, R₄ et R₅, qui sont identiques ou différents les uns des autres, représentent:
   - l'iode,
   - l'hydrogène,
   - COOH,
   - COO⁻M^{y+}, M représentant un cation physiologiquement acceptable et y un nombre entier de 1 à 3,
   - 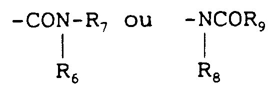 dans lesquels R₆, R₇, R₈ et R₉, qui sont identiques ou différents les uns des autres, représentent l'hydrogène ou un groupe alkyle inférieur linéaire ou ramifié, hydroxyalkyle inférieur linéaire ou ramifié, polyhydroxyalkyle inférieur linéaire ou ramifié, alcoxyalkyle inférieur linéaire ou ramifié, alcoxyhydroxyalkyle inférieur linéaire ou ramifié, alcoxypolyhydroxyalkyle inférieur linéaire ou ramifié,
   les substituants R₂, R₃ et R₄ pouvant également représenter un groupe 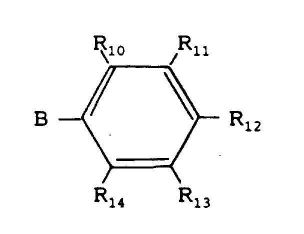 dans lequel
   - les substituants R₁₀, R₁₁, R₁₂, R₁₃ et R₁₄, qui sont identiques ou différents les uns des autres, ont les mêmes significations que R₁, R₂, R₃, R₄ et R₅, et
   - B représente une liaison simple ou un groupe -R₁₅-D-R₁₆- dans lequel R₁₅ et R₁₆, qui sont identiques ou différents, représentent les groupes: 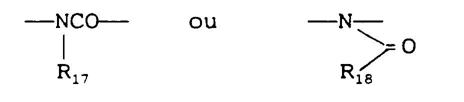 dans lesquels
      - R₁₇ et R₁₈ ont les mêmes significations que R₆ et
      - D représente une liaison simple ou a les mêmes significations que R₆ sauf l'hydrogène,
- A représente un groupe de liaison propre à permettre le greffage d'un dérivé radio-opaque iodé, notamment du dérivé de formule (II), sur les groupes aminés dudit composé macromoléculaire, et
- n représente un nombre entier variant d'environ 3 à environ 500, de préférence d'environ 10 à environ 300 et, plus préférentiellement encore, d'environ 30 à environ 200,
étant entendu qu'au moins l'un des substituants R₁, R₂, R₃, R₄, R₅, R₁₀, R₁₁, R₁₂, R₁₃ et R₁₄ représente un atome d'iode.

Les substituants R₆, R₇, R_{8,} R₉, R₁₇ et R₁₈ sont constitués de préférence par un atome d'hydrogène ou les radicaux suivants:

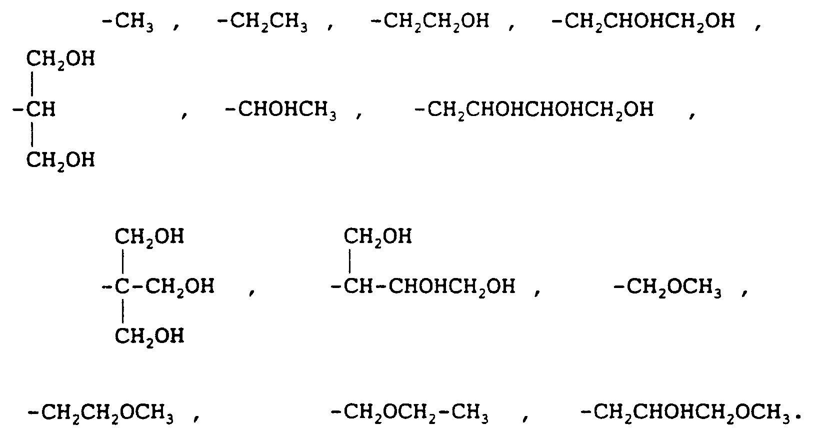

D représente de préférence l'un des radicaux suivants:

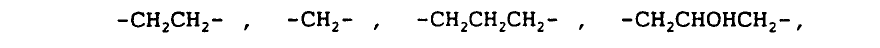

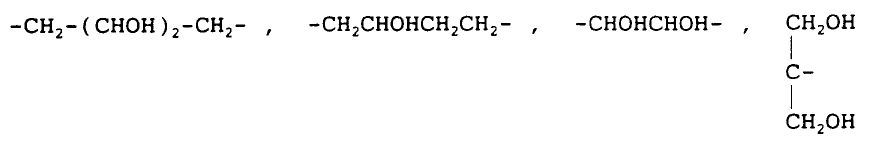

Le composé macromoléculaire polyaminé iodé conforme à l'invention est dit saturé lorsque chacun de ses groupes aminés initialement présents porte un dérivé iodé G et il est dit insaturé lorsqu'après fixation du dérivé G il reste des groupes aminés libres.

Selon un mode de réalisation avantageux du composé macromoléculaire polyaminé iodé conforme à l'invention, le support macromoléculaire polyamine P est choisi dans le groupe comprenant les polyalkylamines, notamment les polyéthylénimines, les polypeptides, notamment la polylysine, la polyornithine, la polyarginine, la polyasparagine, la polyglutamine, les protéines, notamment l'albumine, les polyallylamines de formule générale [-CH₂CH(CH₂NH₂.HCl)-]ₙ, les poly[N(2-aminoéthyl)]méthacrylamides, les polyaminocarbohydrates tels que le poly(aminodextran) et le chitosan.

Parmi les dendrimères utilisables dans le cadre de l'invention et pour reprendre la dénomination utilisée dans les brevets et publications cités dans ce qui précède, on peut citer de façon non limitative :
les dendrimères polyéthylénimines (PEI) de 1ère, 2ème, 3ème, 4ème génération de formule ① et ② ou les polyamidoamines (PAMAM) de 1ère, 2ème, 3ème, 4ème ou 5ème génération de formule ③, ④ et ⑤

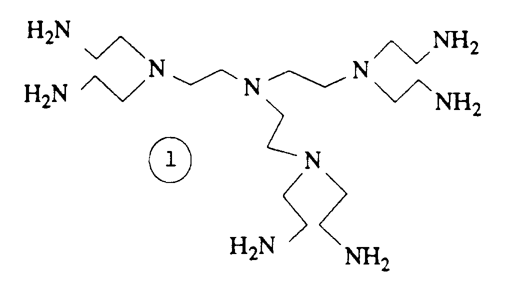

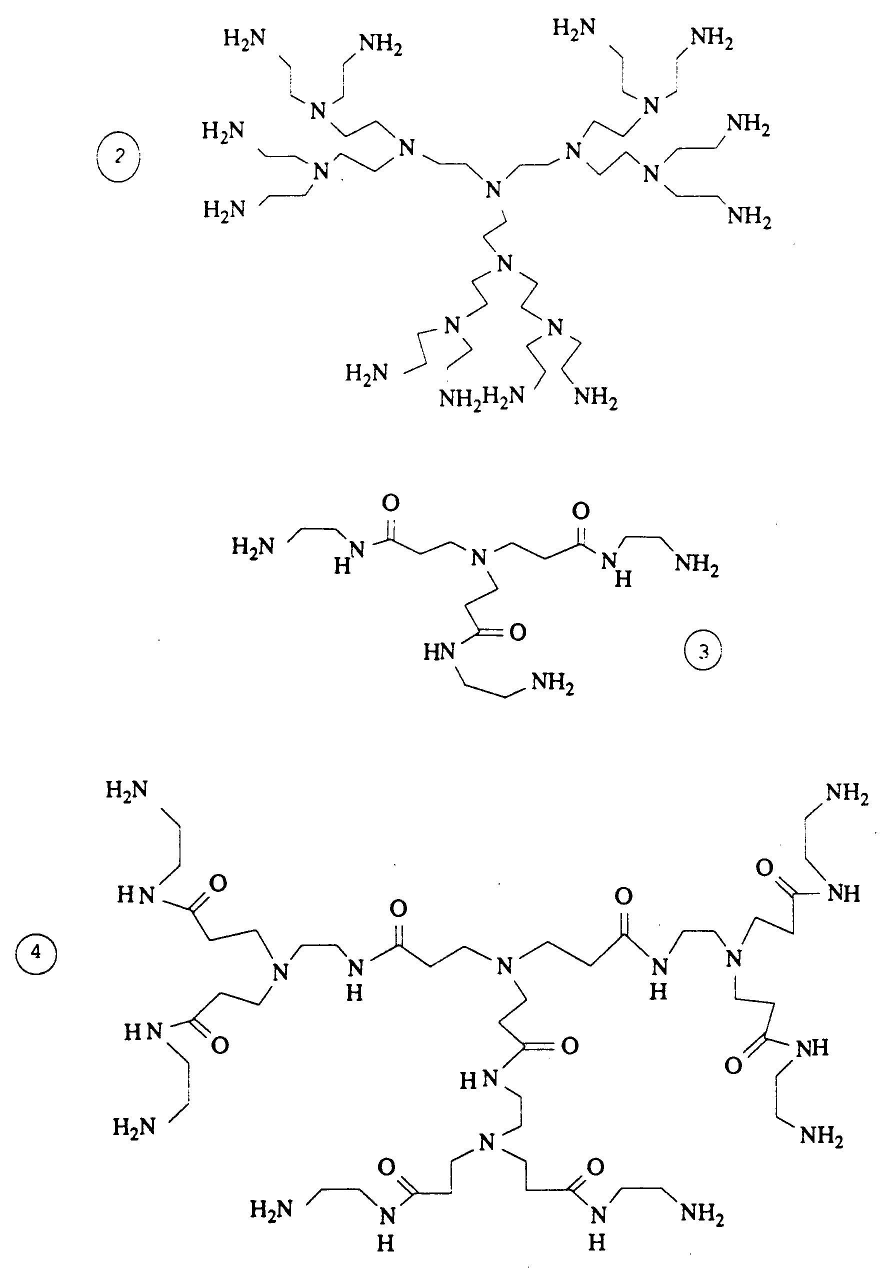

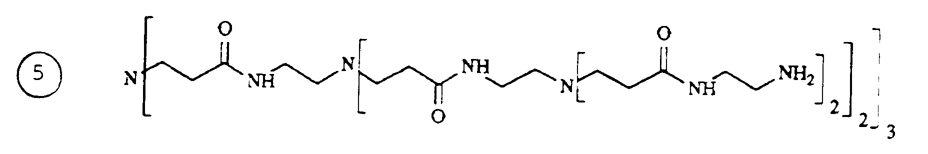

C'est en fonction notamment de l'utilisation diagnostique du produit que l'on choisit le nombre de répétitions de motif unitaire et par là-même le nombre de groupes -NH₂ présents à la périphérie du dendrimère qui détermine le nombre de dérivés radio-opaques polyiodés susceptibles d'être greffés sur le dendrimère.

De la même façon, et ainsi qu'il est décrit dans les brevets et publications cités dans ce qui précède, le motif unitaire est généralement choisi parmi :

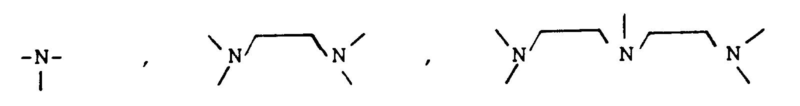

On peut choisir d'autres motifs unitaires permettant la préparation de dendrimères.

Selon un autre mode de réalisation avantageux du composé macromoléculaire polyaminé iodé conforme à l'invention, le groupe de liaison A est choisi parmi les radicaux du groupe comprenant: 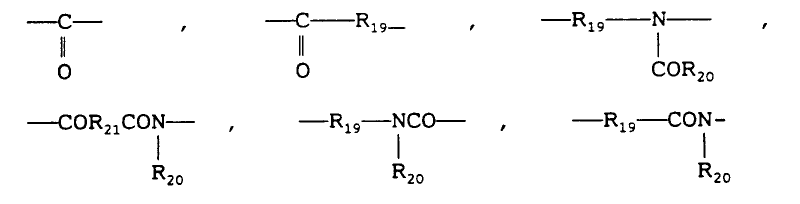 dans lesquels
- R₁₉ représente un groupe alkyle inférieur linéaire ou ramifié, hydroxyalkyle inférieur linéaire ou ramifié, polyhydroxyalkyle inférieur linéaire ou ramifié, alcoxyalkyle inférieur linéaire ou ramifié, alcoxyhydroxyalkyle inférieur linéaire ou ramifié, alcoxypolyhydroxyalkyle inférieur linéaire ou ramifié et
- R₂₀ a les mêmes significations que R₆ et
- R₂₁ représente une liaison simple ou a les mêmes significations que R₁₉.

Le substituant R₂₀ peut avoir les mêmes significations préférentielles que celles indiquées ci-dessus pour R₆, R₇, R₈, R₉, R₁₇ et R₁₈.

Les substituants R₁₉ et R₂₁ sont constitués de préférence par les radicaux suivants:

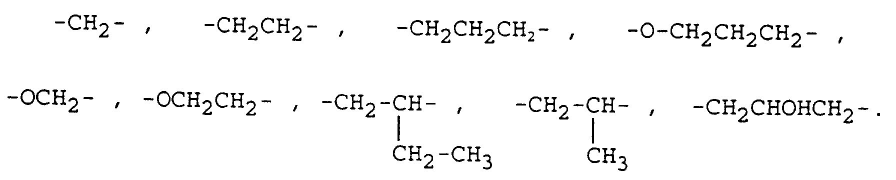

Par groupe alkyle inférieur ou alcoxyalkyle inférieur, on désigne des groupes en C₁ à C₆; par groupe polyhydroxyalkyle ou polyhydroxyalcoxyalkyle, on désigne des groupes portant de 2 à 5 groupes hydroxyle.

Selon un autre mode de réalisation avantageux du composé macromoléculaire polyaminé iodé conforme à l'invention, le dérivé radio-opaque iodé G est un dérivé benzénique de formule: 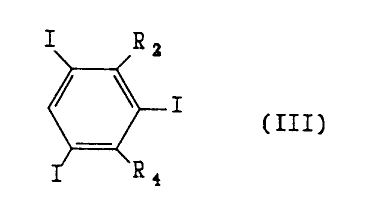 dans laquelle R₂ et R₄ ont les significations données ci-dessus.

Selon un autre mode de réalisation du composé macromoléculaire polyaminé iodé conforme à l'invention, le dérivé radio-opaque iodé est un dérivé benzénique choisi dans le groupe comprenant ceux répondant aux formules: 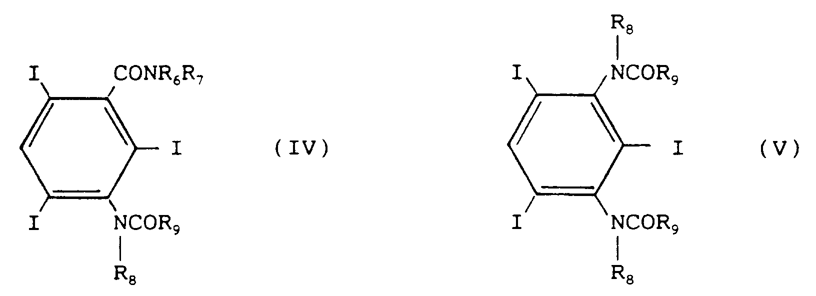 dans lesquelles R₆, R₇, R₈ et R₉ ont les significations données ci-dessus.

Le procédé, conforme à l'invention, pour la préparation du composé macromoléculaire polyaminé iodé conforme à l'invention, est caractérisé par le fait que
- l'on sélectionne un support macromoléculaire polyaminé P et que l'on soumet au moins trois de ses groupes aminés
   - soit à une réaction d'acylation, au sein d'un solvant du groupe comprenant le DMAC, le DMF et le DMSO, éventuellement en présence d'une base minérale ou organique du groupe comprenant la soude, la triéthylamine, le carbonate de sodium, avec un composé de formule:

      Z₁-A-G (VI)

      dans laquelle G est tel que défini plus haut et Z₁-A est choisi parmi: 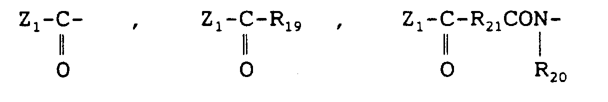 dans lesquels R₁₉, R₂₀, R₂₁ sont tels que définis précédemment et Z₁ représente un atome de chlore ou de brome,
      les groupes hydroxyle éventuellement présents étant préalablement protégés,
   - soit à une réaction d'alkylation, au sein d'un solvant du groupe comprenant le DMAC, le DMF et le DMSO, éventuellement en présence d'une base minérale ou organique du groupe comprenant la soude, la triéthylamine, le carbonate de sodium, avec un composé de formule:

      Z₂-A-G (VII)

      dans laquelle G est tel que défini plus haut et Z₂-A est choisi parmi: 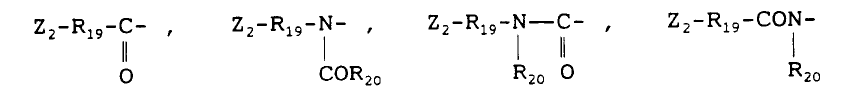 dans lesquels R₁₉, R₂₀ sont tels que définis précédemment et Z₂ représente un atome de chlore, de brome, d'iode, un groupe OMs (O-mésyle), OTs (O-tosyle), ou un époxyde, les groupes hydroxyle éventuellement présents pouvant être préalablement protégés,
une partie au moins des groupes aminés résiduels du support macromoléculaire pouvant être soumise, après déprotection des groupes hydroxyle éventuellement présents, au sein d'un solvant du groupe comprenant le DMAC, le DMF et le DMSO, éventuellement en présence d'une base minérale ou organique du groupe comprenant la soude, la triéthylamine, le carbonate de sodium, à une réaction d'alkylation avec un composé de formule:

L-Y (VIII)

dans laquelle Y a les mêmes significations que Z₂ ou représente un groupe -CO activé sous forme d'halogénure d'acide, d'anhydride ou d'ester et L représente un groupe alkyle inférieur linéaire ou ramifié, hydroxy- ou polyhydroxyalkyle inférieur linéaire ou ramifié, alcoxyalkyle inférieur linéaire ou ramifié, alcoxyhydroxy- ou alcoxypolyhydroxyalkyle inférieur linéaire ou ramifié dont les groupes hydroxyle ont été protégés, les groupes hydroxyle protégés de L pouvant ensuite être déprotégés.

Selon un mode de réalisation avantageux du susdit procédé, on sélectionne le dérivé radio-opaque G parmi les dérivés benzéniques de formule (II) et on soumet au moins trois des groupes aminés du support macromoléculaire aminé de formule P
- soit à la réaction d'acylation avec un composé de formule: 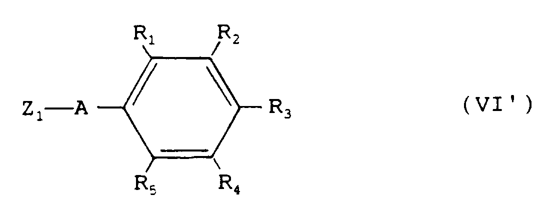
- soit à la réaction d'alkylation avec un composé de formule: 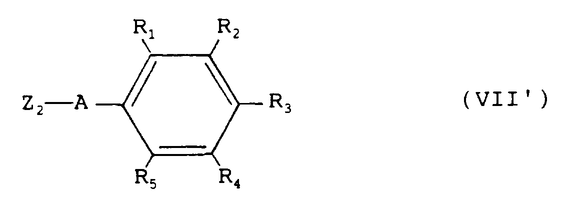 de manière à obtenir le composé de formule (IX) ci-après: 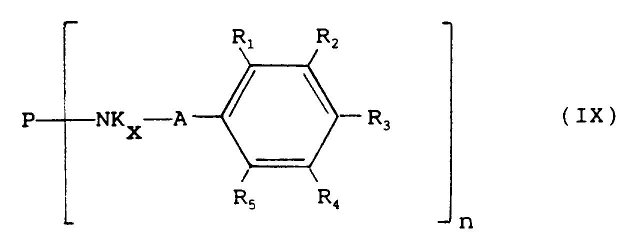 dont au moins une partie des groupes aminés résiduels peut être soumise à la réaction d'alkylation avec un composé L-Y, étant entendu que P, x, n, K, R₁ à R₅, Z₁-A-, Z₂-A-, L et Y ont les significations indiquées plus haut.

Le procédé conforme à l'invention est particulièrement avantageux en raison du fait qu'il n'est pas nécessaire d'activer le support macromoléculaire avant sa réaction avec le dérivé radio-opaque iodé.

Les dérivés polyaminés polyiodés conformes à l'invention peuvent en outre être obtenus par addition de Michael des groupes -NH₂ du dendrimère sur un acrylamide iodé préparé selon le procédé décrit dans le document EP 436 316.

On signale, en rapport avec le support macromoléculaire polyaminé P, que les polyacides aminés et les polyalkylamines sont commercialisés par la Société Sigma Chemical Co. (Strasbourg, France), que les polyallylamines et les polyalkylamines sont commercialisées par la Société Aldrich Chemical Company (Milwaukee, WI) et par la Société Polysciences Inc. (Warrington, PA), que la synthèse des poly[N-(2-aminoéthyl)]méthacrylamides est décrite dans la demande WO 9012050, que le polyaminodextran et sa préparation sont décrits dans le brevet US 4.699.784 et que le chitosan est commercialisé par la Société Sigma Chemical Co.

Les poids moléculaires des supports macromoléculaires retenus dans le cadre de l'invention sont d'environ 5.000 à environ 200.000, de préférence d'environ 5.000 à environ 100.000.

En ce qui concerne les dérivés iodés radio-opaques G de formule (II), on a recours avantageusement à ceux identifiés ci-après, mis en oeuvre sous forme de chlorure d'acide ou d'acide et regroupés en fonction du nombre d'atomes iodés dans la molécule.

Ainsi,
- parmi les composés à un atome d'iode, on peut citer ceux de formules: 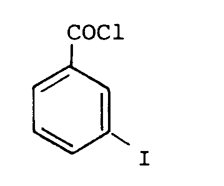 décrit dans Zh. Org. Khim. 19 (11), 2368-73 (1983)
   et 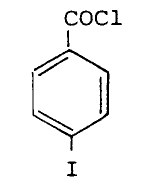 décrit dans Org. Synth. 61, 8-13 (1983),
- parmi les composés à deux atomes d'iode, on peut citer celui de formule: 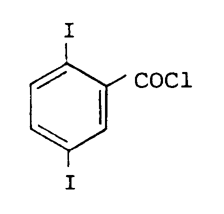 décrit dans Chemical Abstracts 68 (10): 40524j,
- parmi les dérivés à trois atomes d'iode, on peut utiliser ceux déjà connus comme agents de contraste, à savoir:
   - celui connu sous l'appellation diatrizoate et décrit dans le brevet norvégien N° 59-134075,
   - celui connu sous l'appellation ioxithalamate et décrit dans la DE-OS 16 43 440,
   - celui connu sous l'appellation metrizoate et décrit dans la publication "Farmakoterapi" 18:1-29 (1962),
   - celui connu sous l'appellation iothalamate et décrit dans la publication "J. Med. Chem." 6:24-26 (1963),
   - ceux de formules: 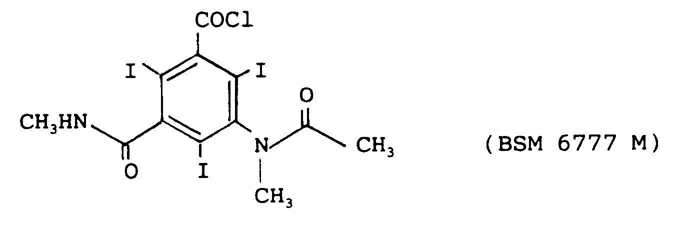 et 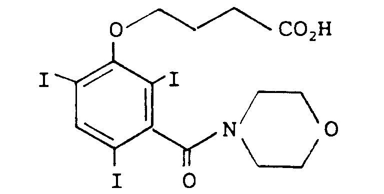 décrit dans Chemical Abstracts 72: 43699p (1970), 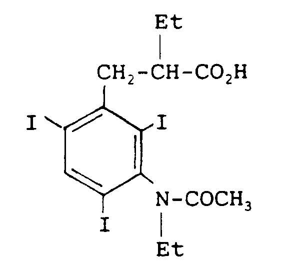 décrit dans Chemical Abstracts 72: 100292b (1970), 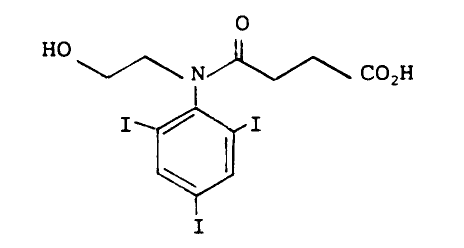 décrit dans Chemical Abstracts 72: 66587q (1970), 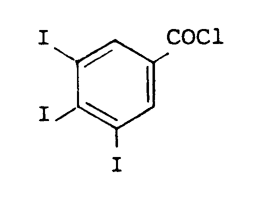 décrit dans Chemical Abstracts 80 (23): 133786j,
- parmi les dérivés comportant quatre atomes d'iode, on peut citer celui de formule: 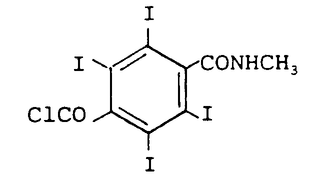 décrit dans Chemical Abstracts 76 (5): 24873g,
- parmi les dérivés comportant cinq atomes d'iode, on peut citer celui de formule: 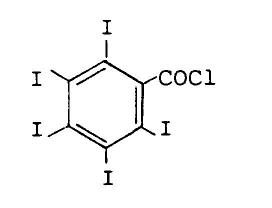 décrit dans Chemical Abstracts 87 (25): 200955y,
- parmi les dérivés comportant six atomes d'iode, on peut citer ceux décrits dans le brevet FR 2 272 640 et dont les formules sont notamment:

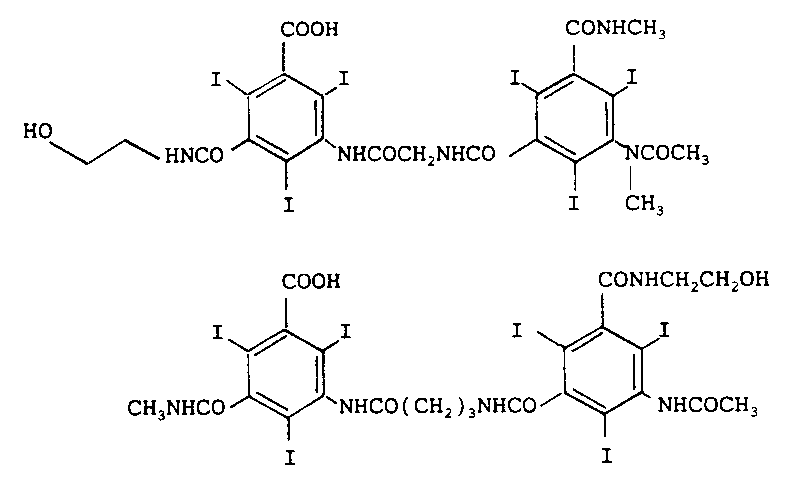

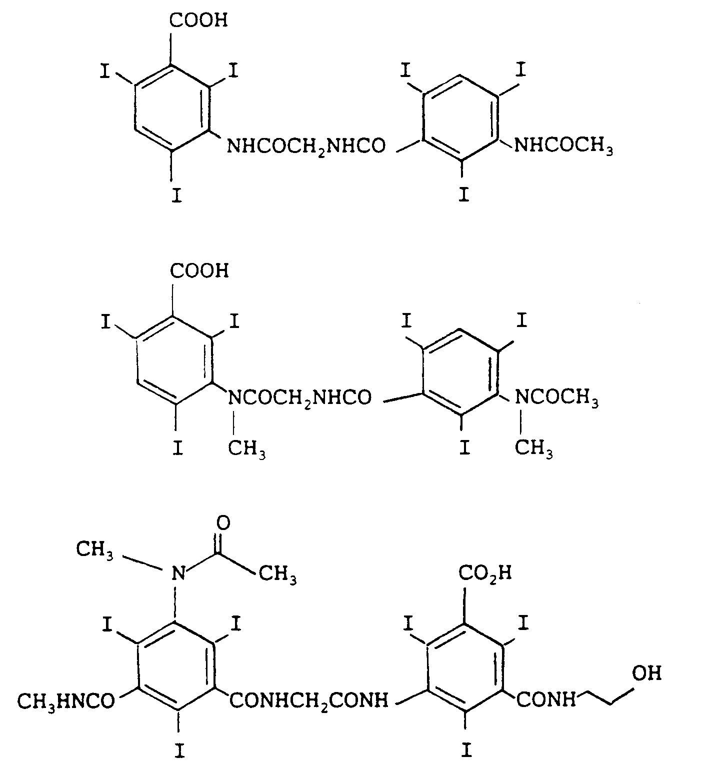

Conformément à l'invention, on a recours non seulement aux dérivés iodés sous forme de mélange racémique mais également aux stéréoisomères tels que les énantiomères, diastéréoisomères, atropoisomères, isomères SYN-ANTI, ENDO-EXO, E-Z, liés à la présence d'atomes de carbone asymétriques et/ou aux empêchements de rotation dus à l'encombrement stérique apporté par les atomes d'iode et/ou par les substituants R₁ à R₂₁.

On signale que, dans le cas des dérivés iodés disponibles sous forme d'acide carboxylique, le passage au chlorure d'acide suivant le schéma: 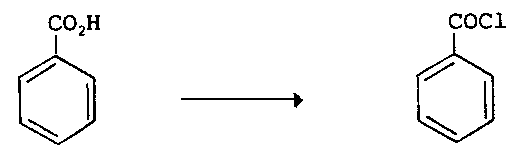 s'effectue par une chloruration en présence de SOCl₂ ou Cl-CO-CO-Cl, de manière classique, les éventuels groupes hydroxyle du dérivé iodé ayant été au préalable protégés, par exemple sous forme d'acétates ou d'acétals cycliques, de façon connue en elle-même.

Le passage à un dérivé iodé sous forme de chlorure d'acide permet ensuite la préparation des polymères conformes à l'invention par mise en oeuvre du procédé conforme à l'invention.

Parmi les composés de formule L-Y mis à réagir avec les groupes aminés du support macromoléculaire n'ayant pas réagi avec le dérivé iodé, on peut citer ceux capables d'apporter des groupements hydrophiles au polymère iodé selon l'invention tels que le chloropropanediol, le glycidol et le chloroéthanol. L'intérêt principal de cette alkylation du support macromoléculaire est qu'elle améliore la solubilité du composé final.

En rapport avec les notions de saturation et d'insaturation rappelés plus haut, on indique que l'invention englobe non seulement les polymères polyaminés iodés du type: 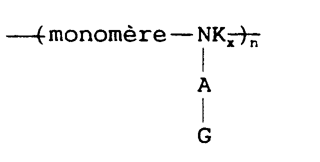 mais aussi ceux de formule:

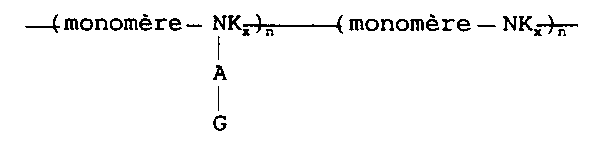

De même, dans la variante selon laquelle les groupes aminés du polymère restant après fixation du dérivé iodé sont alkylés avec un composé L-Y, on peut avoir également les deux types de produits suivants:

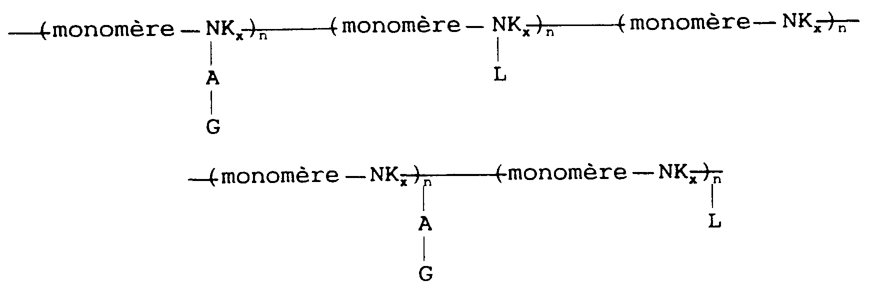

L'invention englobe également les polymères dans lesquels le groupe aminé est situé linéairement dans la chaîne constituant le monomère:

(̵monomère―NHₓ)̵ₙ

et également les polymères dans lesquels le groupe aminé est situé de façon ramifiée dans le monomère:

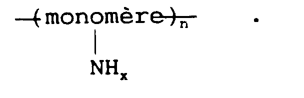

L'invention pourra être encore mieux comprise à l'aide des exemples non limitatifs qui suivent et qui dont donnés en rapport avec des modes de réalisation avantageux.

### EXEMPLE 1

### Préparation du polymère iodé de formule:

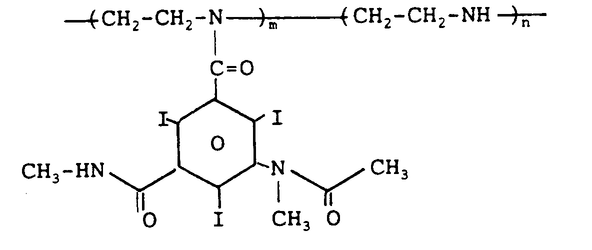

### Synthèse no. 1

On introduit 20 g de polyéthylénimine de poids moléculaire moyen 50.000 (Sigma Chemical Co.) à 50% en solution aqueuse (0,232 m) dans 50 ml de DMAC.

On ajoute par petites portions 50 g du chlorure d'acide 2,4,6-triiodo-3-N-méthylcarbamoyl-5-N-acétyl-N-méthylaminobenzoïque (0,077 m) obtenu selon le procédé décrit dans le brevet français N° 2.272.640.

Le milieu réactionnel, maintenu à la température ambiante, est agité pendant 24 heures, puis est versé dans 2 litres de méthanol.

Le précipité obtenu est filtré, lavé avec de l'éther et séché sous vide.

Après lavage avec de la soude 1 N, on obtient 43 g de produit avec un rendement de 86%.
Pourcentage en iode: 51% (poids/poids).

### Synthèse no. 2

On introduit 20 g de polyéthylénimine de poids moléculaire moyen 50.000 (Sigma Chemical Co.) à 50% en solution aqueuse (0,232 m) dans 50 ml de DMAC.

On ajoute par petites portions, à la température ambiante, 20 g du chlorure d'acide utilisé dans la synthèse no. 1 (0,031 m).

Le milieu réactionnel est agité pendant 24 heures, puis est versé dans 2 litres d'éther. Le précipité obtenu est filtré et séché sous vide.

On obtient 29 g de produit avec un rendement de 90%.
Pourcentage en iode: 41% (poids/poids).

### EXEMPLE 2

### Préparation du polymère iodé de formule:

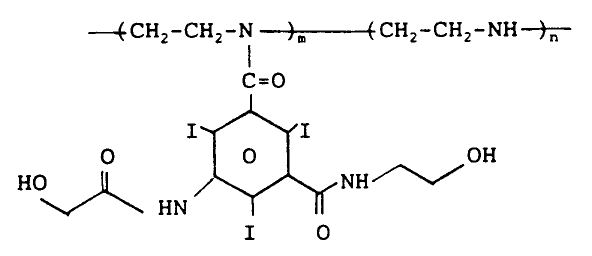

On introduit 20 g de polyéthylénimine de poids moléculaire moyen 50.000 (Sigma Chemical Co.) à 50% en solution aqueuse (0,232 m) dans 50 ml de DMAC.

On ajoute par petites portions, à la température ambiante, 26,5 g de chlorure d'acide 2,4,6-triiodo-3-N-(2-acétoxyéthyl)-5-acétoxyacétamido-isophtalique (0,0348 m) obtenu selon le procédé décrit dans la demande de brevet EP 0 426 610.

Le milieu réactionnel est agité pendant 2 heures, puis est versé dans 2 litres d'éther éthylique.

Après saponification avec de la soude, le polymère iodé obtenu est purifié sur résine IRN 77 et IRA 68.
Pourcentage en iode: 40,5% (poids/poids).

### EXEMPLE 3

### Préparation du polymère iodé de formule:

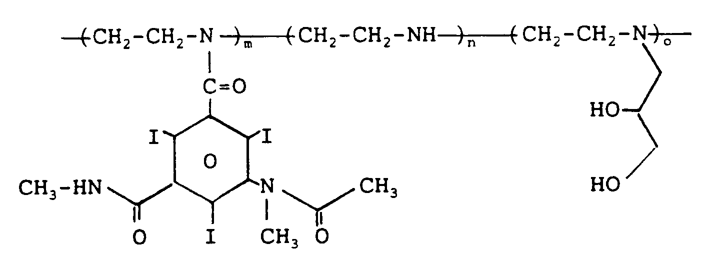

On dissout 5 g du polymère iodé obtenu à l'exemple 1 (synthèse n° 2) dans 50 ml de méthanol.

On ajoute goutte à goutte 2,5 ml de glycidol (Merck) à 45°C.

Le mélange réactionnel est chauffé à 60°C pendant 6 heures.

La solution méthanolique ainsi obtenue est versée dans 500 ml d'éther éthylique.

Le précipité obtenu est lavé dans 200 ml d'éther éthylique et séché sous vide.

On obtient le produit recherché avec un rendement de 92%.
Pourcentage en iode: 29,5% (poids/poids).

### EXEMPLE 4

### Préparation d'un dendrimère polyiodé:

### A. Préparation de la polyamidoamine de formule (d) ci-dessous.

On prépare tout d'abord une polyamidoamine en ayant recours aux étapes 1 à 4.

### Etape 1: Préparation du composé de formule

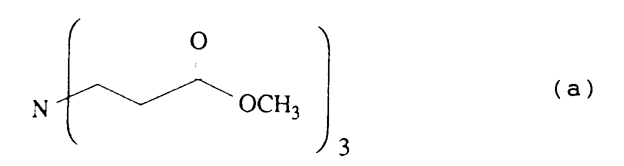

300 g (3,48 moles) d'acrylate de méthyle (disponible commercialement auprès de ALDRICH - Strasbourg) sont mis à réagir dans 100 ml d'une solution d'ammoniac dans le méthanol à 8%, à température ambiante et pendant 3,5 jours, selon la méthode décrite dans le brevet américain US 4 507 466.

On obtient 240 g du produit (a) sous la forme d'une huile jaunâtre.
- CCM: Silice éther de pétrole/éther éthylique 50/50 rf: 0,6
- RMN: ¹H (ppm, 200 mHz) : 3,58 singulets 9 H groupes méthyle; 2,68 triplets 6 H méthylène; 2,40 triplets 6 H méthylène.

### Etape 2: Préparation du composé de formule

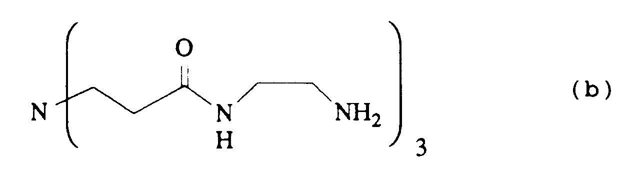

28,1 g (0,102 mole) du produit (a) obtenu à l'étape 1 sont mis à réagir avec 500 g (8,3 moles) d'éthylènediamine dans 270 ml de méthanol pendant deux jours à température ambiante, selon la méthode décrite dans le brevet américain US 4 507 466.

On obtient 36 g du produit (b) sous forme d'une huile jaunâtre (Rendement : 99%)
- RMN: ¹H (ppm), 200 MHz)
1,5 singulet 6 H-NH₂; 2,2 triplets 6 H méthylène; 2,6 multiplets 12 H éthylènediamine; 3,0 multiplets 6 H méthylène; 8,0 triplets NH amide.

### Etape 3: Préparation du composé de formule

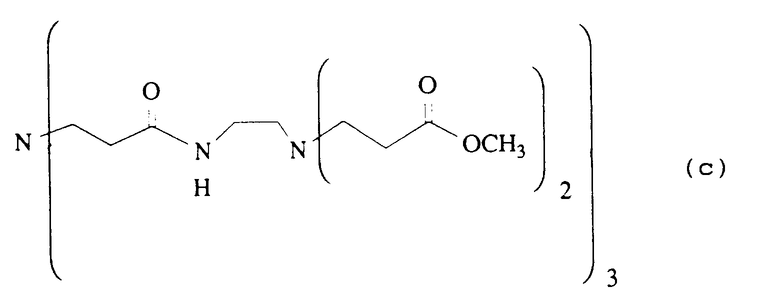

30 g (83,5 mmoles) du produit (b) obtenu à l'étape 2 en solution dans 125 ml de méthanol sont mis à réagir avec 143 g (1,67 mole) d'acrylate de méthyle selon la méthode décrite dans le brevet américain US 4 507 466.

Le mélange est chauffé à 32°C puis est agité à température ambiante pendant 21 heures.

On obtient 65 g de produit (c), soit un rendement de 75%.
- RMN¹³C: (ppm, 50 mHz)
51 méthyle; 171,1 CO amide; 172,4 CO ester.

### Etape 4: Préparation de la polyamidoamine de formule

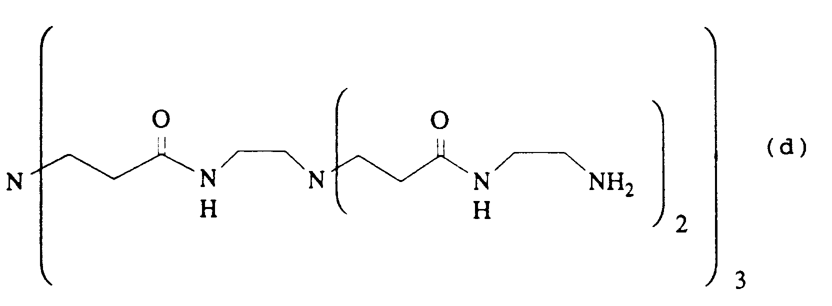

65 g (74,3 mmoles) du produit (c) obtenu à l'étape 3 dans 100 ml de méthanol sont mis à réagir avec 622 g (10,3 moles) d'éthylènediamine dans 500 ml de méthanol selon la méthode décrite dans le brevet américain US 4 507 466.

Après trois jours de réaction à température ambiante et évaporation sous pression réduite on obtient 76 g du produit (d) (Rendement: 98%) sous forme d'une huile jaunâtre.
- RMN¹³C: (ppm, 50 mHz)
171,7 et 171,1 CO amide.

### B. Préparation du dendrimère polyiodé

2 g (0,00192 mole) du produit (d) obtenu à l'étape 4 sont dissous dans 50 ml de méthanol. 1,9 ml (0,0135 mole) de triéthylamine sont ajoutés au milieu réactionnel. 8,7 g (0,0135 mole) du chlorure de 5-Nméthylacétamido-2,4,6-triiodo-3N-méthylaminocarbonylbutamide préparé selon la méthode décrite dans le brevet français FR 2 272 640 sont ajoutés sous forme de poudre dans le milieu réactionnel. L'agitation est maintenue pendant 24 heures à température ambiante. La solution méthanolique est versée sur 500 ml d'éther éthylique.

Le précipité obtenu est filtré et séché. Le produit est purifié par chromatographie sur plaque de silice. 9,5 g de produit brut sont obtenus sous forme d'une poudre blanche.
Pourcentage en iode: 35% (poids/poids).
CCM (SiO₂) dichlorométhane/méthanol 50/50.
Rf: 0,46.

### EXEMPLE 5

### Préparation du polymère iodé de formule

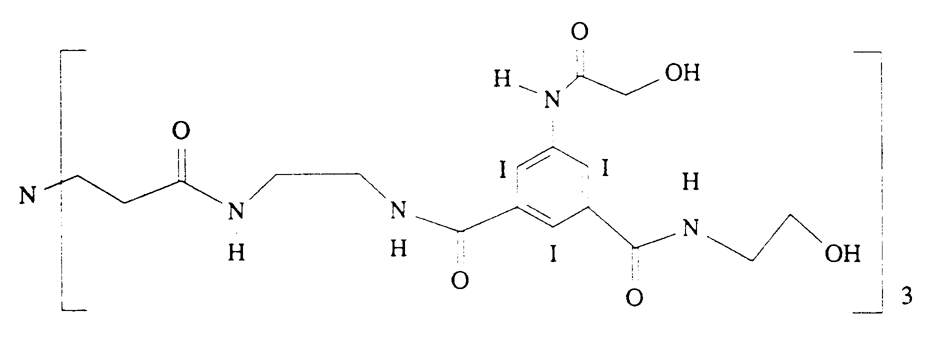

1,8 g (5 mmoles) du produit obtenu à l'exemple 4 étape 2 sont dissous dans 50 ml de N,N-diméthylacétamide. 2,8 ml (20 mmoles) de triéthylamine sont ajoutés au milieu réactionnel.

12,2 g (16 mmoles) de chlorure d'acide 2,4,6-triiodo-3-N-(2-acétoxyéthyl)-5-acétoxyacétamido-isophtalique obtenu selon le procédé décrit dans la demande de brevet européen EP 426 610 sont ajoutés goutte à goutte sous forme de poudre dans le milieu réactionnel. L'agitation est maintenue pendant douze heures à température ambiante. Les sels de triéthylamine sont éliminés par filtration et la solution est versée sur 400 ml d'éther éthylique.

Le précipité obtenu est repris dans 300 ml de méthanol et est saponifié avec 5 g (36,2 mmoles) de K₂CO₃ pendant 24 heures.

Après purification, 7 g de poudre blanche sont obtenus, soit un rendement de 61%.

| | |
|---|---|
| Pourcentage en iode | Trouvé : 48,8% |
| | Théorie: 50%. |
| Pureté: 97,8%. | |

### EXEMPLE 6

### Préparation du polymère iodé de formule

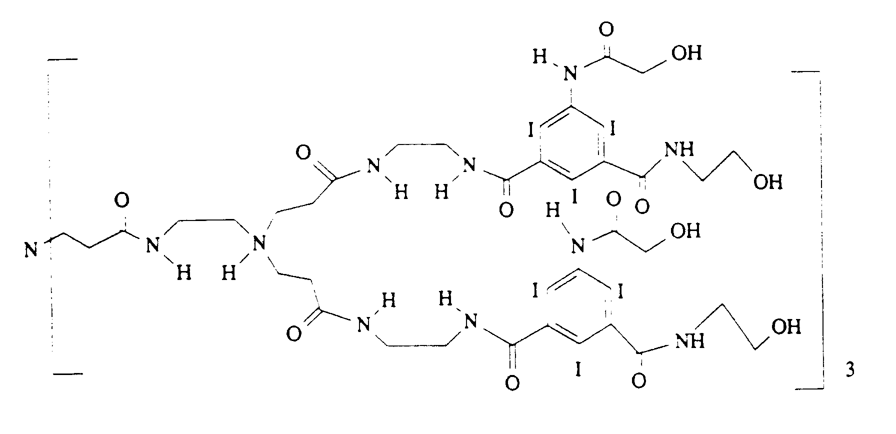

5 g (4,8 mmoles) du produit obtenu à l'exemple 4 étape 4 sont dissous dans 150 ml de N,N-diméthylacétamide. 4,7 ml (33,6 mmoles) de triéthylamine sont ajoutés au milieu réactionnel.

25,6 g (33,6 mmoles) de chlorure d'acide 2,4,6-triiodo-3-N-(2-acétoxyéthyl)-5-acétoxyacétamido-isophtalique sont ajoutés goutte à goutte sous forme de poudre dans le milieu réactionnel. L'agitation est maintenue pendant 24 heures à température ambiante. Les sels de triéthylamine sont éliminés par filtration et la solution est versée sur 400 ml d'éther éthylique.

Le précipité obtenu est repris dans 300 ml de méthanol et est saponifié avec 10 g (0,072 mole) de K₂CO₃.

Après purification, 13 g de poudre blanche sont obtenus, soit un rendement de 58%.

| | |
|---|---|
| Pourcentage en iode | Trouvé : 45,4% |
| | Théorie: 46,7%. |
| Pureté: 97,2%. | |

Les composés macromoléculaires polyaminés iodés conformes à l'invention présentent des caractéristiques particulières qui font leur intérêt en tant qu'agents de contraste en radiologie aux rayons X, notamment une excellente stabilité et une concentration en atomes d'iode radio-opaques pouvant être particulièrement élevée.

La composition conforme à l'invention à base de ces agents de contraste est constituée par une solution aqueuse ou par une suspension d'au moins un agent selon l'invention, de préférence dans de l'eau bidistillée.

La composition de diagnostic notamment pour rayons X, utilisable chez l'homme ou l'animal, conforme à l'invention est caractérisée par le fait qu'elle comprend une quantité efficace d'au moins l'un des composés macromoléculaires polyaminés iodés conformes à l'invention, la quantité efficace étant celle qui permet d'injecter une solution aqueuse du polymère comprenant de préférence d'environ 5 à environ 50 g d'iode/100 ml de solution.

Les compositions en question peuvent contenir les additifs habituellement utilisés dans les compositions pharmaceutiques à base de produits de contraste, à savoir notamment:
- le chlorure de sodium,
- le chlorhydrate de tris(hydroxyméthyl)amino-méthane,
- l'amine de tris(hydroxyméthyl)amino-méthane,
- l'héparine,
- le citrate de sodium et
- le calciédétate de sodium.

Dans le cadre de sa mise en oeuvre en radiologie aux rayons X chez l'homme, cette composition est administrée à une dose utile d'environ 10 à environ 250 ml de solution aqueuse contenant d'environ 30 à environ 500 g d'iode par kg.

Son administration, lorsqu'elle se présente sous la forme de solutions aqueuses, peut se faire par les voies entérale ou parentérale, notamment par les voies orale, rectale, intraveineuse, intra-articulaire, intra-artérielle, sous-arachnoïdienne ainsi que par les voies bronchique, lymphatique et intra-utérine.

Lorsqu'elle se présente sous la forme d'une suspension dans l'eau, la composition conforme à l'invention peut être administrée par les voies entérale, orale, rectale ou bronchique.

Le pH des susdites solutions est ajusté à une valeur physiologiquement acceptable, généralement comprise entre environ 6,5 et environ 7,5.

Dans certaines applications particulières, il peut être nécessaire, pour faire le diagnostic d'une pathologie donnée, notamment au niveau d'un organe spécifique, d'avoir recours à ce que l'on appelle une "vectorisation" de l'agent de contraste qui peut être obtenue par encapsulation dudit agent dans des liposomes, ou par la fixation d'une biomolécule, notamment des anticorps poly- et monoclonaux.

Les pathologies dont le diagnostic peut être facilité par une telle vectorisation sont notamment des pathologies tumorales, les pathologies du foie et les anomalies de distribution du volume sanguin.

## Revendications

1. Composé macromoléculaire polyaminé d'origine biologique ou synthétique, éventuellement type dendrimère, caractérisé par le fait qu'il porte au moins trois dérivés radio-opaques iodés.

2. Composé macromoléculaire polyaminé iodé selon la revendication 1, caractérisé par le fait qu'il présente la formule générale: 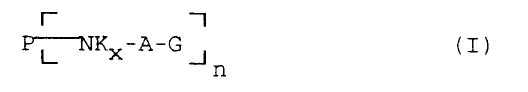 dans laquelle
- P représente le reste d'un composé ou support macromoléculaire polyaminé d'origine biologique ou synthétique,
- N représente un atome d'azote,
- K représente un atome d'hydrogène ou un groupe alkyle linéaire ou ramifié en C₁ à C₆, hydroxy- ou polyhydroxyalkyle inférieur linéaire ou ramifié, alcoxyalkyle linéaire ou ramifié en C₁ à C₆, alcoxyhydroxy- ou alcoxypolyhydroxyalkyle inférieur linéaire ou ramifié, K pouvant également représenter un groupe -A-G,
- x est un nombre entier égal à 0 ou 1,
- G est un dérivé radio-opaque iodé constitué notamment par un dérivé benzénique de formule: 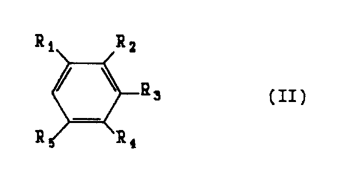 dans laquelle les constituants R₁, R₂, R₃, R₄ et R₅, qui sont identiques ou différents les uns des autres, représentent:
- l'iode,
- l'hydrogène,
- COOH,
- COO⁻M^{y+}, M représentant un cation physiologiquement acceptable et y un nombre entier de 1 à 3,
- 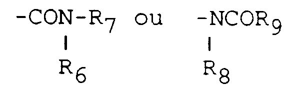 dans lesquels R₆, R₇, R₈ et R₉, qui sont identiques ou différents les uns des autres, représentent l'hydrogène ou un groupe alkyle linéaire ou ramifié en C₁ à C₆, hydroxyalkyle inférieur linéaire ou ramifié, polyhydroxyalkyle inférieur linéaire ou ramifié, alcoxyalkyle linéaire ou ramifié en C₁ à C₆, alcoxyhydroxyalkyle inférieur linéaire ou ramifié, alcoxypolyhydroxyalkyle inférieur linéaire ou ramifié,
les substituants R₂, R₃ et R₄ pouvant également représenter un groupe 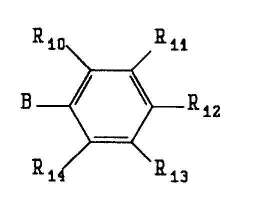 dans lequel
- les substituants R₁₀, R₁₁, R₁₂, R₁₃ et R₁₄, qui sont identiques ou différents les uns des autres, ont les mêmes significations que R₁, R₂, R₃, R₄ et R₅, et
- B représente une liaison simple ou un groupe -R₁₅-D-R₁₆- dans lequel R₁₅ et R₁₆, qui sont identiques ou différents, représentent les groupes: 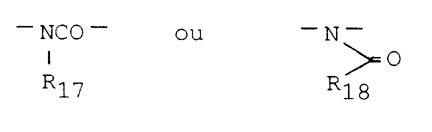 dans lesquels
- R₁₇ et R₁₈ ont les mêmes significations que R₆ et
- D représente une liaison simple ou a les mêmes significations que R₆ sauf l'hydrogène,
- A représente un groupe de liaison propre à permettre le greffage d'un dérivé radio-opaque iodé, notamment du dérivé de formule (II), sur les groupes aminés dudit composé macromoléculaire, et
- n représente un nombre entier variant d'environ 3 à environ 500, de préférence d'environ 10 à environ 300 et, plus préférentiellement encore, d'environ 30 à environ 200,
étant entendu qu'au moins l'un des substituants R₁, R₂, R₃, R₄, R₅, R₁₀, R₁₁, R₁₂, R₁₃ et R₁₄ représente un atome d'iode.

3. Composé macromoléculaire polyaminé iodé selon la revendication 2, caractérisé par le fait que les substituants R₆, R₇, R_{8,} R₉, R₁₇ et R₁₈ sont constitués par un atome d'hydrogène ou les radicaux suivants:

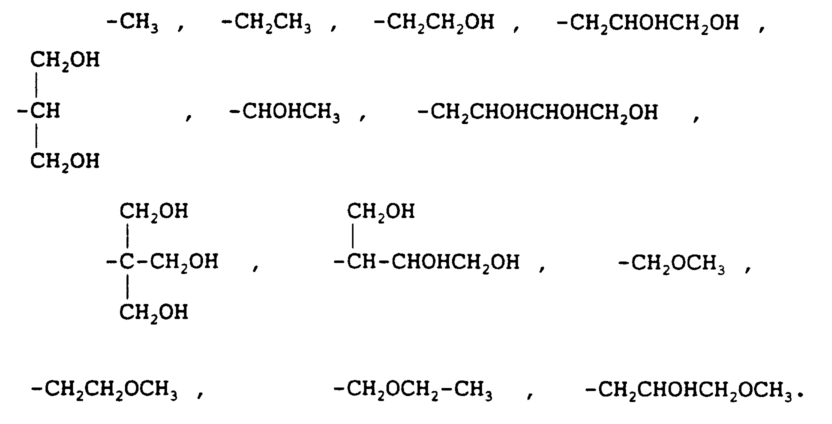

4. Composé macromoléculaire polyaminé iodé selon l'une des revendications 2 et 3, caractérisé par le fait que D représente l'un des radicaux suivants:

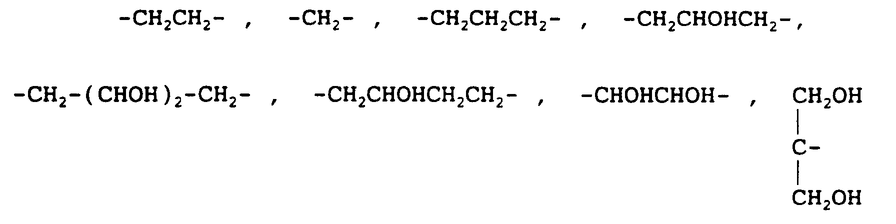

5. Composé macromoléculaire polyaminé iodé selon l'une des revendications 2 à 4, caractérisé par le fait que le support macromoléculaire polyaminé P est choisi dans le groupe comprenant les polyalkylamines, notamment les polyéthylénimines, les polypeptides, notamment la polylysine, la polyornithine, la polyarginine, la polyasparagine, la polyglutamine, les protéines, notamment l'albumine, les polyallylamines de formule générale [-CH₂CH(CH₂NH₂.HCl)-]ₙ, les poly[N(2-aminoéthyl)]méthacrylamides, les polyaminocarbohydrates tels que le poly(aminodextran) et le chitosan.

6. Composé macromoléculaire polyaminé iodé de type dendrimère selon l'une des revendications 2 à 4, caractérisé par le fait que le support macromoléculaire polyaminé P est choisi dans le groupe comprenant les polyalkylamines, notamment les polyéthylénimines, les polyamidoamines, les polylysines ramifiées, les arborols.

7. Composé macromoléculaire polyaminé iodé selon l'une des revendications 2 à 6, caractérisé par le fait que le groupe de liaison A est choisi parmi les radicaux du groupe comprenant: 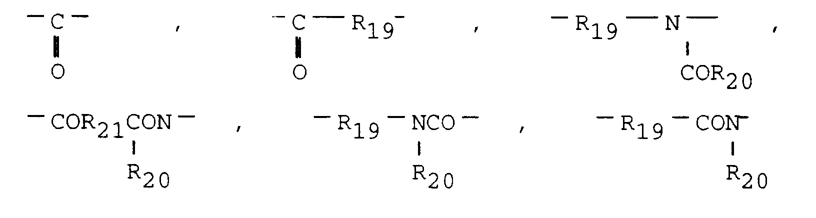 dans lesquels
- R₁₉ représente un groupe alkyle linéaire ou ramifié en C₁ à C₆, hydroxyalkyle inférieur linéaire ou ramifié, polyhydroxyalkyle inférieur linéaire ou ramifié, alcoxyalkyle linéaire ou ramifié en C₁ à C₆, alcoxyhydroxyalkyle inférieur linéaire ou ramifié, alcoxypolyhydroxyalkyle inférieur linéaire ou ramifié et
- R₂₀ a les mêmes significations que R₆ et
- R₂₁ représente une liaison simple ou a les mêmes significations que R₁₉.

8. Composé macromoléculaire polyaminé iodé selon l'une des revendications 2 à 7, caractérisé par le fait que le dérivé radio-opaque iodé est constitué par l'un des dérivés benzéniques de formules:

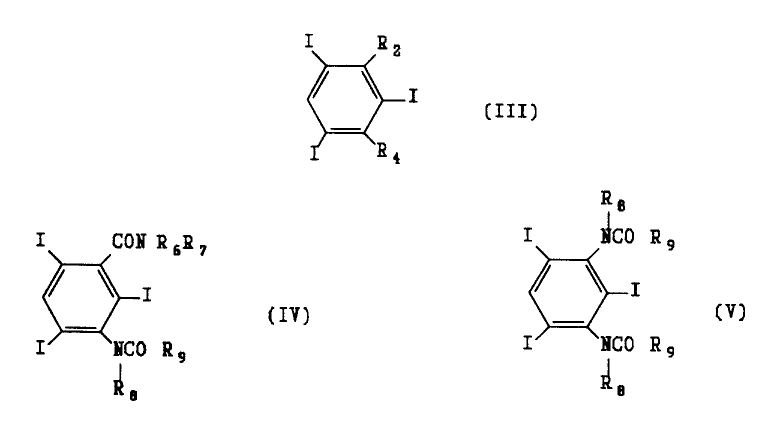

9. Composé macromoléculaire polyaminé iodé selon l'une des revendications 2 à 7, caractérisé par le fait qu'il présente la formule: 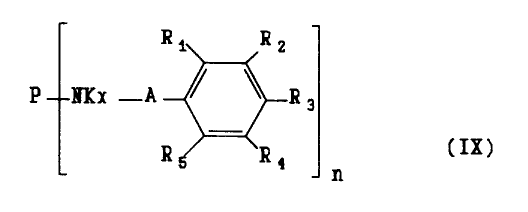 dont au moins une partie des groupes aminés résiduels est éventuellement soumise à la réaction d'alkylation avec un composé de formule L-Y dans laquelle Y représente un atome de chlore, de brome, d'iode, un groupe OMs (O-mésyle), OTs (O-tosyle), ou un époxyde, les groupes hydroxyle éventuellement présents pouvant être préalablement protégés, ou encore un groupe -CO activé sous forme d'halogénure d'acide, d'anhydride ou d'ester et L représente un groupe alkyle linéaire ou ramifié en C₁ à C₆, hydroxy- ou polyhydroxyalkyle inférieur linéaire ou ramifié, alcoxyalkyle linéaire ou ramifié en C₁ à C₆, alcoxy-hydroxy- ou alcoxypolyhydroxyalkyle inférieur linéaire ou ramifié dont les groupes hydroxyle ont été protégés, les groupes hydroxyle protégés de L pouvant ensuite être déprotégés.

10. Procédé pour la préparation d'un composé macromoléculaire polyaminé iodé selon l'une des revendications 1 à 9, caractérisé par le fait que
- l'on sélectionne un support macromoléculaire polyaminé P et que l'on soumet au moins trois de ses groupes aminés
- soit à une réaction d'acylation, au sein d'un solvant du groupe comprenant le DMAC, le DMF et le DMSO, éventuellement en présence d'une base minérale ou organique du groupe comprenant la soude, la triéthylamine, le carbonate de sodium, avec un composé de formule:
Z₁-A-G (VI)
dans laquelle G est tel que défini plus haut et Z₁-A est choisi parmi: 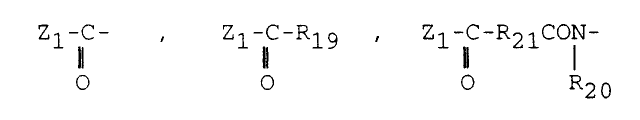 dans lesquels R₁₉, R₂₀, R₂₁ sont tels que définis précédemment et Z₁ représente un atome de chlore ou de brome,
les groupes hydroxyle éventuellement présents étant préalablement protégés,
- soit à une réaction d'alkylation, au sein d'un solvant du groupe comprenant le DMAC, le DMF et le DMSO, éventuellement en présence d'une base minérale ou organique du groupe comprenant la soude, la triéthylamine, le carbonate de sodium, avec un composé de formule:
Z₂-A-G (VII)
dans laquelle G est tel que défini plus haut et Z₂-A est choisi parmi: 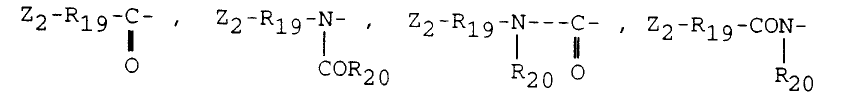 dans lesquels R₁₉, R₂₀ sont tels que définis précédemment et Z₂ représente un atome de chlore, de brome, d'iode, un groupe OMs (O-mésyle), OTs (O-tosyle), ou un époxyde, les groupes hydroxyle éventuellement présents pouvant être préalablement protégés,
une partie au moins des groupes aminés résiduels du support macromoléculaire pouvant être soumise, après déprotection des groupes hydroxyle éventuellement présents, au sein d'un solvant du groupe comprenant le DMAC, le DMF et le DMSO, éventuellement en présence d'une base minérale ou organique du groupe comprenant la soude, la triéthylamine, le carbonate de sodium, à une réaction d'alkylation avec un composé de formule:
L-Y (VIII)
dans laquelle Y a les mêmes significations que Z₂ ou représente un groupe -CO activé sous forme d'halogénure d'acide, d'anhydride ou d'ester et L représente un groupe alkyle linéaire ou ramifié en C₁ à C₆, hydroxy- ou polyhydroxyalkyle inférieur linéaire ou ramifié, alcoxyalkyle linéaire ou ramifié en C₁ à C₆, alcoxyhydroxy- ou alcoxypolyhydroxyalkyle inférieur linéaire ou ramifié dont les groupes hydroxyle ont été protégés, les groupes hydroxyle protégés de L pouvant ensuite être déprotégés.

11. Composition de diagnostic pour rayons X, utilisable chez l'homme ou l'animal, caractérisée par le fait qu'elle comprend une quantité efficace d'au moins l'un des composés macromoléculaires polyaminés iodés selon l'une des revendications 1 à 9.

12. Composition de diagnostic pour rayons X, utilisable chez l'homme ou l'animal, caractérisée par le fait qu'elle comprend au moins un dendrimère polyaminé polyiodé comportant au moins trois dérivés radio-opaques iodés selon l'une des revendications 1 à 9.

## Claims

1. Macromolecular polyamine compound of biological or synthetical origin, possibly of the dendrimer-type, characterized by the fact that it comprises at least three iodine-containing radio-opaque derivatives.

2. Macromolecular polyamine iodine-containing compound according to claim 1, characterized by the fact that it presents the general formula:
P⁅NKₓ-A-G]ₙ (I)
wherein
- P represents the radical of a macromolecular polyamine compound or support of biological or synthetical origin,
- N represents a nitrogen atom,
- K represents a hydrogen atom or a linear or branched alkyl group in C₁ to C₆, a lower linear or branched hydroxy- or polyhydroxy-alkyl group, a linear or branched alkoxyalkyl group in C₁ to C₆, a lower linear or branched alkoxyhydroxy- or alkoxypolyhydroxyalkyl group, as well as a group -A-G,
- x is an integer equal to 0 or 1,
- G is an iodine-containing radio-opaque derivative consisting in particular of a benzenic derivative of the formula: 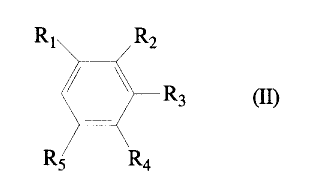 wherein the constituents R₁, R₂, R₃, R₄ and R₅, which are identical or different from one another, represent:
- iodine,
- hydrogen,
- COOH,
- COO⁻M^{y+}, M representing a physiologically acceptable cation and y an integer from 1 to 3,
- 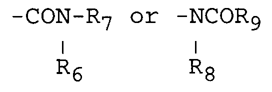 wherein R₆, R₇, R₈ and R₉, which are identical or different from one another, represent hydrogen or a linear or branched alkyl group in C₁ to C₆, a lower linear or branched hydroxyalkyl group, a lower linear or branched polyhydroxyalkyl group, a linear or branched alkoxyalkyl group in C₁ to C₆, a lower linear or branched alkoxyhydroxyalkyl group, a lower linear or branched alkoxypolyhydroxyalkyl group,
the substituents R₂, R₃ and R₄ representing possibly also a group 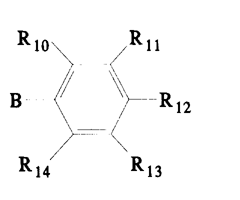 wherein
- the substituents R₁₀, R₁₁, R₁₂, R₁₃ and R₁₄, which are identical or different from one another, have the same significations as R₁, R₂, R₃, R₄ and R₅, and
- B represents a single bond or a group -R₁₅-D-R₁₆-wherein R₁₅ and R₁₆, which are identical or different, represent the groups: 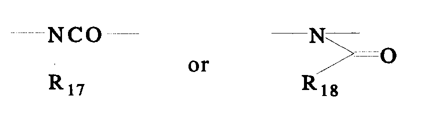 wherein
- R₁₇ and R₁₈ have the same significations as R₆ and
- D represents a single bond or has the same significations as R₆ except hydrogen,
- A represents a bonding group adapted to allow the grafting of an iodine-containing radio-opaque derivative, in particular of the derivative of formula (II), on the amine groups of said macromolecular compound, and
- n represents an integer varying from about 3 to about 500, preferably from about 10 to about 300 and, still more preferably, from about 30 to about 200,
with the proviso that at least one of the substituents R₁, R₂, R₃, R₄, R₅, R₁₀, R₁₁, R₁₂, R₁₃ and R₁₄ represents an iodine atom.

3. Macromolecular polyamine iodine-containing compound according to claim 2, characterized by the fact that the substituents R₆, R₇, R₈, R₉, R₁₇ and R₁₈ are consisting of a hydrogen atom or of the following radicals:

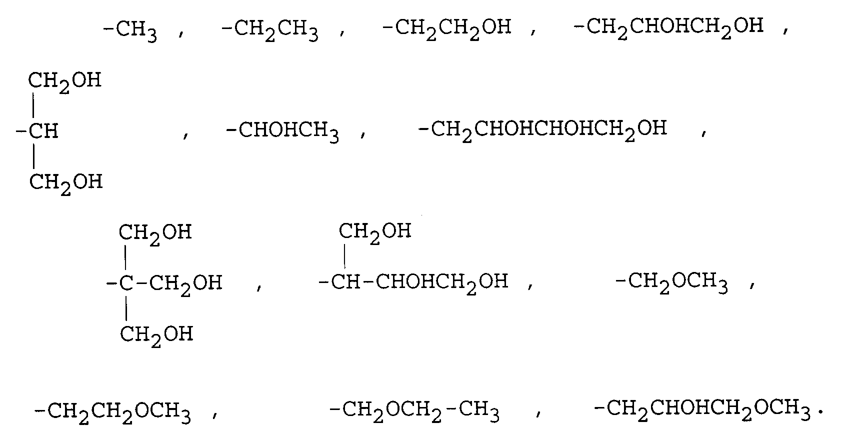

4. Macromolecular polyamine iodine-containing compound according to one of claims 2 and 3, characterized by the fact that D represents one of the following radicals:

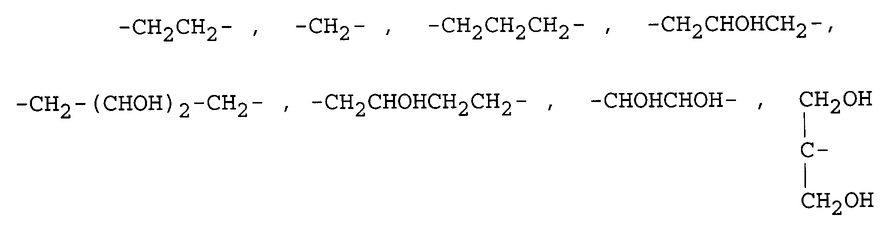

5. Macromolecular polyamine iodine-containing compound according to one of claims 2 to 4, chararacterized by the fact that the macromolecular polyamine support P is selected from the group comprising the polyalkylamines, in particular the polyethylenimines, the polypeptides, in particular polylysine, polyornithine, polyarginine, polyasparagine, polyglutamine, the proteins, in particular albumine, the polyallylamines of general formula [-CH₂CH(CH₂NH₂.HCl)-]ₙ, the poly[N(2-aminoethyl)]methacrylamides, the polyaminocarbohydrates such as poly(amino-dextran) and chitosan.

6. Macromolecular polyamine iodine-containing compound of the dendrimer-type according to one of claims 2 to 4, characterized by the fact that the macromolecular polyamine support P is selected from the group comprising polyalkylamines,in particular polyethylenimines, polyamidoamines, branched polylysines, arborols.

7. Macromolecular polyamine iodine-containing compound according to one of claims 2 to 6, characterized by the fact that the bonding group A is selected from among the radicals of the group comprising: 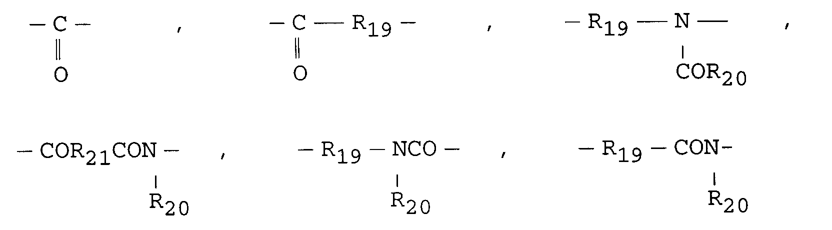 wherein
- R₁₉ represents a linear or branched alkyl group in C₁ to C₆, a lower linear or branched hydroxyalkyl group, a lower linear or branched polyhydroxyalkyl group, a linear or branched alkoxyalkyl group in C₁ to C₆, a lower linear or branched alkoxyhydroxyalkyl group, a lower linear or branched alkoxypolyhydroxyalkyl group and
- R₂₀ has the same significations as R₆ and
- R₂₁ represents a single bond or has the same significations as R₁₉.

8. Macromolecular polyamine iodine-containing compound according to one of claims 2 to 7, characterized by the fact that the radio-opaque iodine-containing derivative is consisting of one of the benzenic derivatives of formulae:

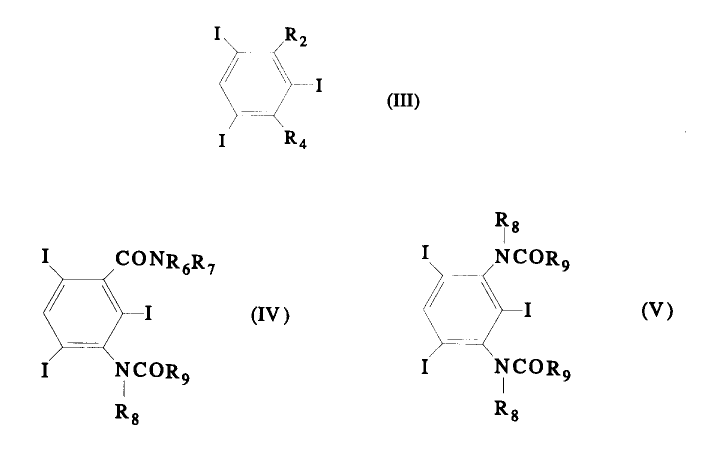

9. Macromolecular polyamine iodine-containing compound according to one of claims 2 to 7, characterized by the fact that it presents the formula: 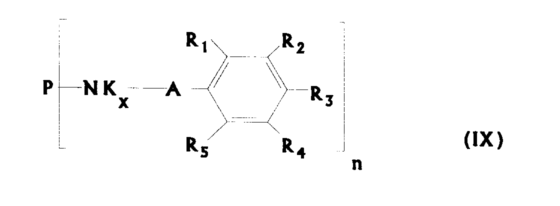 of which at least part of the residual amine groups are possibly subjected to the alkylation reaction with a compound of formula L-Y in which Y represents a chlorine, bromine or iodine atom, an OMs (O-mesyl), OTs (O-tosyl) group, or an epoxyde, the hydroxyl groups which are possibly present being possibly previously protected, or still an activated -CO group in the form of an acid halogenide, an anhydride or an ester and L represents a linear or branched alkyl group in C₁ to C₆, a lower linear or branched hydroxy- or polyhydroxyalkyl group, a linear or branched alkoxyalkyl group in C₁ to C₆, a lower linear or branched alkoxyhydroxy- or alkoxypolyhydroxyalkyl group, the hydroxyl groups of which have been protected, it being possible to then deprotect the protected hydroxyl groups of L.

10. Process for the preparation of a macromolecular polyamine iodine-containing compound according to one of claims 1 to 9, characterized by the fact that a macromolecular polyamine support P is selected and that at least three of its amine groups are subjected
- either to an acylation reaction, within a solvent of the group comprising DMAC, DMF and DMSO, possibly in the presence of a mineral or organic base of the group comprising NaOH, triethylamine, sodium carbonate, with a compound of the formula:
Z₁-A-G (VI)
wherein G is such as hereabove defined and Z₁-A is selected from among: 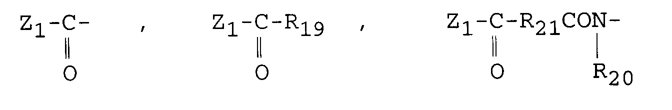 wherein R₁₉, R₂₀, R₂₁ are such as hereabove defined and Z₁ represents a chlorine or bromine atom,
the hydroxyl groups which are possibly present being previously protected,
- or to an alkylation reaction, within a solvent of the group comprising DMAC, DMF and DMSO, possibly in the presence of a mineral or organic base of the group comprising NaOH, triethylamine, sodium carbonate, with a compound of formula:
Z₂-A-G (VII)
wherein G is such as hereabove defined and Z₂-A is selected from among: 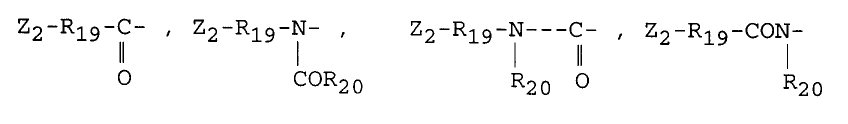 wherein R₁₉, R₂₀ are such as previously defined and Z₂ represents a chlorine, bromine or iodine atom, an OMs (O-mesyl), OTs (O-tosyl) group, or an epoxyde, the hydroxyl groups which are possibly present being possibly previously protected,
at least part of the residual amine groups of the macromolecular support being possibly subjected, after deprotection of the hydroxyl groups which are possibly present, within a solvent of the group comprising DMAC, DMF and DMSO, possibly in the presence of a mineral or organic base of the group comprising NaOH, triethylamine, sodium carbonate, to an alkylation reaction with a compound of the formula:
L-Y (VIII)
wherein Y has the same significations as Z₂ or represents an activated -CO group in the form of an acid halogenide, an anhydride or an ester and L represents a linear or branched alkyl group in C₁ to C₆, a lower linear or branched hydroxy- or polyhydroxyalkyl group, a linear or branched alkoxyalkyl group in C₁ to C₆, a lower linear or branched alkoxyhydroxy- or alkoxypolyhydroxyalkyl group, the hydroxyl groups of which have been protected, it being possible to then deprotect the protected hydroxyl groups of L.

11. Diagnosis composition for X-rays, adapted to be used in human or animal, characterized by the fact that it comprises an efficient amount of at least one of the macromolecular polyamine iodine-containing compounds according to one of claims 1 to 9.

12. Diagnosis composition for X-rays, adapted to be used in human or animal, characterized by the fact that it comprises at least one polyamine polyiodine-containing dendrimer comprising at least three iodine-containing radio-opaque derivatives according to one of claims 1 to 9.

## Patentansprüche

1. Polyaminierte makromolekulare Verbindung biologischer oder synthetischer Herkunft, gegebenenfalls vom Dendrimertyp, dadurch gekennzeichnet, daß sie mindestens drei iodierte radiopake Derivate trägt.

2. Iodierte polyaminierte makromolekulare Verbindung nach Anspruch 1, dadurch gekennzeichnet, daß sie die allgemeine Formel aufweist:
P⁅NKₓ-A-G]ₙ (I)
bei der
- P für den Rest einer polyaminierten makromolekularen Verbindung biologischer oder synthetischer Herkunft bzw. eines solchen Trägers steht,
- N für ein Stickstoffatom steht,
- K für ein Wasserstoffatom oder eine lineare oder verzweigte C₁ bis C₆-Alkylgruppe, eine lineare oder verzweigte niedere Hydroxy- oder Polyhydroxyalkylgruppe, eine lineare oder verzweigte C₁ bis C₆-Alkoxyalkylgruppe, eine lineare oder verzweigte niedere Alkoxyhydroxy- oder Alkoxypolyhydroxyalkylgruppe steht, wobei K ebenso für eine Gruppe -A-G stehen kann,
- x eine ganze Zahl gleich 0 oder 1 ist,
- G ein iodiertes radiopakes Derivat ist, welches insbesondere aus einem Benzolderivat mit der Formel 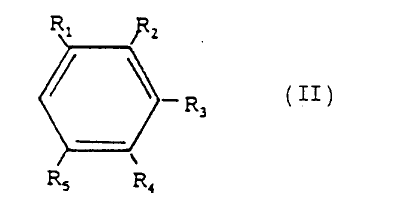 besteht, bei dem die Bestandteile R₁, R₂, R₃, R₄ und R₅, die gleich oder voneinander verschieden sind, repräsentieren:
- Iod,
- Wasserstoff,
- COOH,
- COO⁻M^{y+}, wobei M für ein physiologisch akzeptables Kation und y für eine ganze Zahl von 1 bis 3 steht,
- 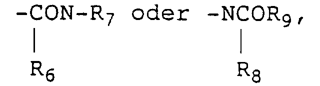 wobei R₆, R₇, R₈ und R₉, die gleich oder voneinander verschieden sind, für Wasserstoff oder eine lineare oder verzweigte C₁ bis C₆-Alkylgruppe, eine lineare oder verzweigte niedere Hydroxyalkylgruppe, eine lineare oder verzweigte niedere Polyhydroxyalkylgruppe, eine lineare oder verzweigte C₁ bis C₆-Alkoxyalkylgruppe, eine lineare oder verzweigte niedere Alkoxyhydroxyalkylgruppe, eine lineare oder verzweigte niedere Alkoxypolyhydroxyalkylgruppe stehen,
die Substituenten R₂, R₃ und R₄ ebenso für eine Gruppe 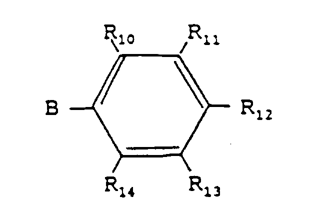 stehen können, bei der
- die Substituenten R₁₀, R₁₁, R₁₂, R₁₃ und R₁₄, die gleich oder voneinander verschieden sind, die gleichen Bedeutungen wie R₁, R₂, R₃, R₄ und R₅ haben, und
- B für eine einfache Bindung oder eine Gruppe -R₁₅-D-R₁₆- steht, bei der R₁₅ und R₁₆, die gleich oder verschieden sind, für die Gruppen: 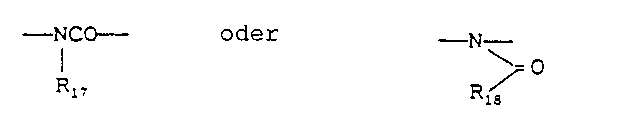 stehen, bei denen
- R₁₇ und R₁₈ die gleichen Bedeutungen wie R₆ haben, und
- D für eine einfache Bindung steht oder die gleichen Bedeutungen wie R₆ mit der Ausnahme von Wasserstoff hat,
- A für eine Bindungsgruppe steht, die dazu geeignet ist, das Aufpropfen eines iodierten radiopaken Derivates, insbesondere des Derivates mit der Formel (II), auf die aminierten Gruppen der makromolekularen Verbindung zu ermöglichen, und
- n für eine ganze Zahl steht, die von ca. 3 bis ca. 500, bevorzugt von ca. 10 bis ca. 300 und insbesondere bevorzugt von ca. 30 bis ca. 200 variiert,
mit der Bedingung, daß mindestens einer der Substituenten R₁, R₂, R₃, R₄, R₅, R₁₀, R₁₁, R₁₂, R₁₃ und R₁₄ für ein Iodatom steht.

3. Iodierte polyaminierte makromolekulare Verbindung nach Anspruch 2, dadurch gekennzeichnet, daß die Substituenten R₆, R₇, R₈, R₉, R₁₇ und R₁₈ aus einem Wasserstoffatom oder den folgenden Resten bestehen:

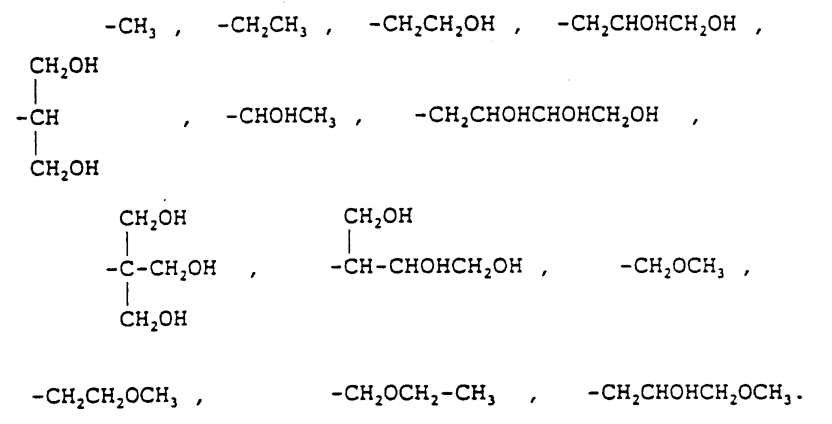

4. Iodierte polyaminierte makromolekulare Verbindung nach einem der Ansprüche 2 und 3, dadurch gekennzeichnet, daß D für eines der folgenden Radikale steht:

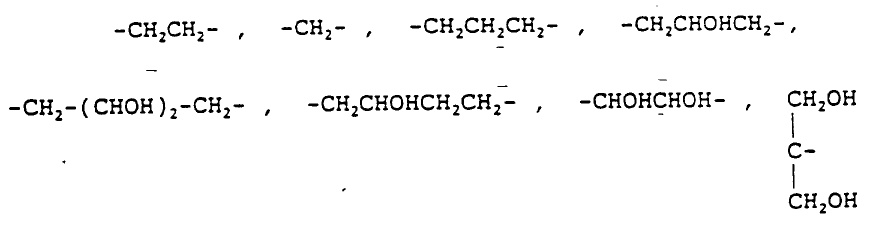

5. Iodierte polyaminierte makromolekulare Verbindung nach einem der Ansprüche 2 bis 4, dadurch gekennzeichnet, daß der polyaminierte makromolekulare Träger P aus der Gruppe ausgewählt ist, welche aufweist: Polyalkylamine, insbesondere Polyethylenimine, Polypeptide, insbesondere Polylysin, Polyornithin, Polyarginin, Polyasparagin, Polyglutamin, Proteine, insbesondere Albumin, Polyallylamine der allgemeinen Formel [-CH₂CH(CH₂NH₂.HCl)-]ₙ, Poly[N(2-aminoethyl)]methacrylamide, Polyaminokohlenhydrate wie z.B. Poly(aminodextran) und Chitosan.

6. Iodierte polyaminierte makromolekulare Verbindung vom Dendrimertyp nach einem der Ansprüche 2 bis 4, dadurch gekennzeichnet, daß der polyaminierte makromolekulare Träger P aus der Gruppe ausgewählt ist, welche aufweist: Polyalkylamine, insbesondere Polyethylenimine, Polyamidoamine, verzweigte Polylysine, Arborole.

7. Iodierte polyaminierte makromolekulare Verbindung nach einem der Ansprüche 2 bis 6, dadurch gekennzeichnet, daß die Bindungsgruppe A ausgewählt ist unter den Resten der Gruppe, welche aufweist: 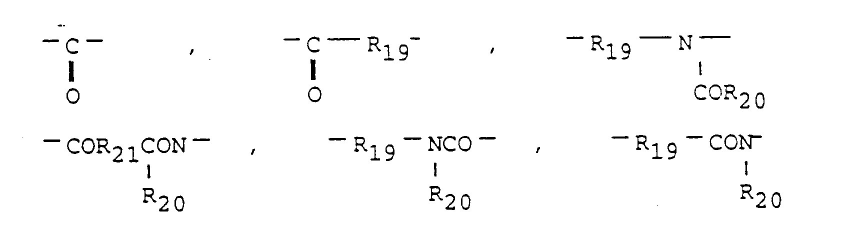 bei denen
- R₁₉ für eine lineare oder verzweigte C₁ bis C₆-Alkylgruppe, eine lineare oder verzweigte niedere Hydroxyalkylgruppe, eine lineare oder verzweigte niedere Polyhydroxyalkylgruppe, eine lineare oder verzweigte C₁ bis C₆-Alkoxyalkylgruppe, eine lineare oder verzweigte niedere Alkoxyhydroxyalkylgruppe, eine lineare oder verzweigte niedere Alkoxypolyhydroxyalkylgruppe steht, und
- R₂₀ die gleichen Bedeutungen hat wie R₆, und
- R₂₁ für eine einfache Bindung steht oder die gleichen Bedeutungen wie R₁₉ hat.

8. Iodierte polyaminierte makromolekulare Verbindung nach einem der Ansprüche 2 bis 7, dadurch gekennzeichnet, daß das iodierte radiopake Derivat aus einem der Benzolderivate mit der Formel: 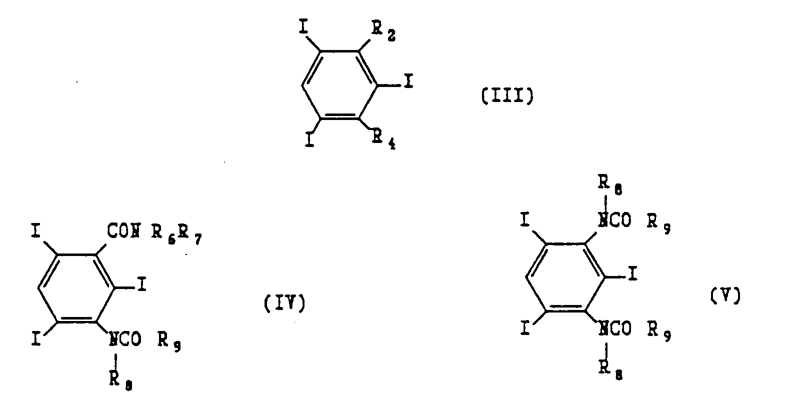 besteht.

9. Iodierte polyaminierte makromolekulare Verbindung nach einem der Ansprüche 2 bis 7, dadurch gekennzeichnet, daß sie die Formel: 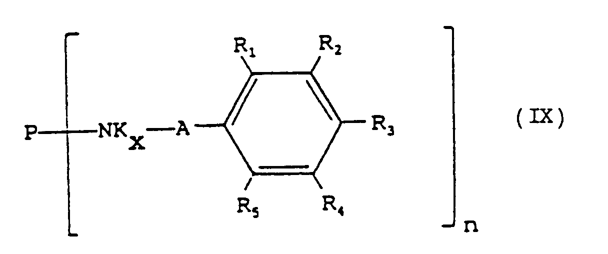 aufweist, bei der mindestens ein Teil der aminierten Restgruppen gegebenenfalls der Alkylierungsreaktion mit einer Zusammensetzung der Formel L-Y unterzogen wird, bei der Y für ein Chlor-, Brom-, Iodatom, eine OMs (O-Mesyl)-, OTs (O-Tosyl)-gruppe oder ein Epoxid steht, wobei die gegebenenfalls vorhandenen Hydroxylgruppen vorausgehend mit einer Schutzgruppe versehen worden sein können, oder auch eine aktivierte -CO-Gruppe in Form eines Säure-, Anhydrid- oder Esterhalogenids, und L für eine lineare oder verzweigte C₁ bis C₆-Alkylgruppe, eine lineare oder verzweigte niedere Hydroxy- oder Polyhydroxyalkylgruppe, eine lineare oder verzweigte C₁ bis C₆-Alkoxyalkylgruppe, eine lineare oder verzweigte niedere Alkoxyhydroxy- oder Alkoxypolyhydroxyalkylgruppe steht, bei denen die Hydroxylgruppen mit einer Schutzgruppe versehen sind und die Schutzgruppe der geschützten Hydroxylgruppen von L anschließend entfernt worden sein kann.

10. Verfahren zur Herstellung einer iodierten polyaminierten makromolekularen Verbindung nach einem der Ansprüche 1 bis 9, dadurch gekennzeichnet, daß
- ein polyaminierter makromolekularer Träger P ausgewählt wird, und daß mindestens drei der aminierten Gruppen davon unterzogen werden:
- entweder eine Acylierungsreaktion in einem Lösungsmittel der Gruppe, welche DMAC, DMF und DMSO aufweist, gegebenenfalls in Gegenwart einer mineralischen oder organischen Base der Gruppe, welche Soda, Triethylamin, Natriumcarbonat aufweist, mit einer Verbindung mit der Formel:
Z₁-A-G (VI),
bei der G der obenstehenden Definition entspricht und Z₁-A ausgewählt ist unter: 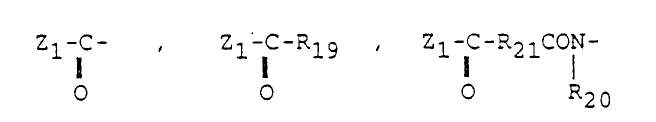 bei denen R₁₉, R₂₀, R₂₁ der obenstehenden Definition entsprechen und Z₁ für ein Chlor- oder Bromatom steht,
die gegebenenfalls vorhandenen Hydroxylgruppen vorausgehend mit einer Schutzgruppe versehen wurden,
- oder eine Alkylierungsreaktion in einem Lösungsmittel der Gruppe, welche DMAC, DMF und DMSO aufweist, gegebenenfalls in Gegenwart einer mineralischen oder organischen Base der Gruppe, welche Soda, Triethylamin, Natriumcarbonat aufweist, mit einer Verbindung mit der Formel:
Z₂-A-G (VII),
bei der G der obenstehenden Definition entspricht und Z₂-A ausgewählt ist unter: 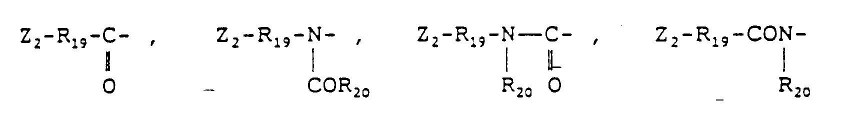 bei denen R₁₉, R₂₀ der obenstehenden Definition entsprechen und Z₂ für ein Chlor-, Brom-, Iod-atom, eine OMs (O-Mesyl)-, OTs (O-Tosyl)-Gruppe oder ein Epoxid steht, wobei die gegebenenfalls vorhandenen Hydroxylgruppen vorausgehend mit einer Schutzgruppe versehen worden sein können,
wobei zumindest ein Teil der aminierten Restgruppen des makromolekularen Trägers nach dem Entfernen der Schutzgruppen der gegebenenfalls vorhandenen Hydroxylgruppen in einem Lösungsmittel der Gruppe, welche DMAC, DMF und DMSO aufweist, gegebenenfalls in Gegenwart einer mineralischen oder organischen Base der Gruppe, welche Soda, Triethylamin, Natriumcarbonat aufweist, einer Alkylierungsreaktion mit einer Verbindung der Formel:
L-Y VIII),
unterzogen werden kann, bei der Y die gleichen Bedeutungen wie Z₂ hat oder für eine aktivierte -CO-Gruppe in Form eines Säure-, Anhydrid- oder Esterhalogenids steht, und L für eine lineare oder verzweigte C₁ bis C₆-Alkylgruppe, eine lineare oder verzweigte niedere Hydroxy- oder Polyhydroxyalkylgruppe, eine lineare oder verzweigte C₁ bis C₆-Alkoxyalkylgruppe, eine lineare oder verzweigte niedere Alkoxyhydroxy- oder Alkoxypolyhydroxyalkylgruppe steht, bei denen die Hydroxylgruppen mit einer Schutzgruppe versehen sind, wobei die Schutzgruppen der Hydroxylgruppen von L anschließend entfernt werden können.

11. Zusammensetzung für die Röntgendiagnostik zur Anwendung beim Menschen oder beim Tier, dadurch gekennzeichnet, daß sie eine wirksame Menge von mindestens einer der iodierten polyaminierten makromolekularen Verbindungen nach einem der Ansprüche 1 bis 9 aufweist.

12. Zusammensetzung für die Röntgendiagnostik zur Anwendung beim Menschen oder beim Tier, dadurch gekennzeichnet, daß sie mindestens ein polyiodiertes polyaminiertes Dendrimer aufweist, welches mindestens drei iodierte radiopake Derivate nach einem der Ansprüche 1 bis 9 aufweist.
